# EUROPEAN PATENT APPLICATION

(11) **EP 1 707 196 A1**
(43) Date of publication of application: **04.10.2006**
(21) Application number: 05258102.2
(22) Date of filing: 29.12.2005
(51) Int. Cl.: A61K 31/216, A61K 47/10

(54) **Improved formulations of fenofibrate**

(30) Priority: 30.03.2005 US 666192 P
(71) Applicant: TEVA PHARMACEUTICAL INDUSTRIES LTD., 49131 Petah Tiqva (IL)
(72) Inventor: Flashner-Barak, Moshe, Petach Tikva 49313 (IL); Lerner, E. Itzhak, Petach Tikva 49541 (IL)
(74) Representative: Gallagher, Kirk James

(57) **Abstract**

The invention provides at least a composition for the treatment of elevated levels of triglycerides, comprising a therapeutically effective amount of fenofibrate or another fibrate drug intimately associated with menthol or a surfactant mixture, wherein the menthol can be menthol or menthol surfactant mixture, and wherein the surfactant mixture can be a mixture of a polyethylene glycol and a poloxamer such as PGE 1000 and poloxamer 407. The invention also provides a method for the treatment of elevated levels of triglycerides in a subject, comprising administering to the subject a composition comprising a therapeutically effective amount of fenofibrate or another fibrate drug and menthol or a surfactant mixture, wherein the fenofibrate or the other fibrate drug is in intimate association with the menthol or surfactant mixture, wherein the menthol can be menthol or menthol surfactant mixture, and wherein the surfactant mixture can be a mixture of a polyethylene glycol and a poloxamer such as PGE 1000 and poloxamer 407.

## Description

### Background

Fenofibrate, (2-[4-(4-chlorobenzoyl) phenoxy]-2-methyl-propanoic acid, 1-methylethyl ester) is one of the fibrate class of drug. It is available as both capsules and tablets. Fenofibrate is apparently a prodrug. The active moiety is reportedly the metabolite fenofibric acid which is reported to be produced in the body by esterases. When dosing fenofibrate, apparently no intact fenofibrate is found in the plasma (Physician's Desk Reference 58th ed. 2004 pages 522 - 525 (PDR)).

Fenofibrate is a very poorly soluble drug. Despite its poor solubility, it is reported to be well absorbed when dosed in the "fed state" and less so in the "fasted state". It is unclear what the bioavailability of the fenofibric acid really is, since much of it is understood to be metabolized to the glucuronide in both presystemic and first pass sites. The absolute bioavailability of fenofibrate cannot supposedly be determined since the compound is insoluble in media suitable for intravenous injection. Following oral administration in healthy volunteers, approximately 60% of a single dose of radiolabelled fenofibrate appeared in urine, primarily as fenofibric acid and its glucuronide conjugate, and 25% was excreted in the feces. (PDR) The absorption of fenofibrate is understood to be increased when administered with food. With fenofibrate tablets, the extent of absorption is increased by approximately 35% when dosed with food (PDR, Martindale 33rd ed. Page 889).

Much effort has been expended to improve the formulation of fenofibrate. US Patent Nos. 4,895,726 and 5,880,148 disclose co-micronizing the fenofibrate with surface active agents. US Patent Nos. 6,074,670 , 6,277,405 and others disclose micronized fenofibrate coated onto hydrosoluble carriers with optional surface active agents. US Patent No. 6,814,977 discloses fenofibrate dissolved in a medium chain glycerol ester of fatty acid, US Patent No. 6,719,999 discloses fenofibrate dissolved in glycerin, propylene glycol, or dimethylisosorbide and US Patent No. 5,827,536 discloses fenofibrate dissolved in diethyleneglycol monoethyl ether.

Several patents disclose specific formulations of micronized fenofibrate with specific polymeric or surface active agent additives while several others describe emulsions and suspensions. US Patent Application Publication No. 20040087656 discloses fenofibrate of particle size less than 2000 nm with an improved bioavailability. US Patent Application Publication No. 20030224059 discloses microparticles of active pharmaceutical ingredients, drug delivery vehicles comprising same, and methods for making them. The disclosure of US20030224059 is incorporated herein by reference in its entirety.

US Patent Application Publication No. 20040198646 discloses compositions comprising solutions of drugs in menthol, especially drugs that are poorly soluble in water, and to methods for making such compositions. The disclosure of US 20040198646 is incorporated in its entirety by reference.

Micronization of the drug and the addition of surface active agents have moderately raised the bioavailability of fenofibrate allowing the amount of drug dosed to be reduced from 100 mg per dose to 67 mg per dose and then subsequently to 54 mg per dose, all with the same bioavailability in the fed state. Nanoparticle formulations of the drug have further allowed the reduction of the dose to 48 mg per dose with the bioavailability of the "fasted state" being reported as similar to the fed state. There is still room for much improvement since it is posited that the true bioavailability of fenofibrate is still relatively low.

The current inventors have surprisingly found that a composition of fenofibrate dissolved in menthol and comprising surface active agents gives a much enhanced bioavailability well beyond anything previously disclosed. The inventors have also surprisingly found formulations with or without menthol of increased solubility and drug release of fenofibrate

### Summary of the Invention

The present invention encompasses a composition for the treatment of elevated levels of triglycerides that comprises a therapeutically effective amount of fenofibrate or another fibrate drug that is intimately associated with menthol. The intimate association may be in the form of a solution of the fenofibrate or other fibrate in menthol but would encompass compositions where at least part of the drug has come out of such a solution due to a process that induces the precipitation of the drug, e.g. saturation such as reducing the volume of the solvent or cooling. The composition may be optionally absorbed in, or adsorbed on a solid carrier by methods exemplified by the teachings in US 2003-0224059 and US 2004-0198646.

The present invention encompasses a composition for the treatment of elevated levels of triglycerides that comprises a therapeutically effective amount of fenofibrate or another fibrate drug that is dissolved in menthol and further comprises at least one surface active agent. The composition may be optionally absorbed on a solid carrier.

The present invention encompasses a composition that comprises a therapeutically effective amount of fenofibrate or another fibrate drug that is dissolved in menthol and further comprises at least one surface active agent. The composition can have a dissolution property in that, when tested in 50 ml 0.1 N HCl at 37°C and 150 rpm, at least about 10%, 30% or 80% of the fenofibrate or the other fibrate drug dissolved in 15 minutes. The composition can also have a dissolution property in that, when tested in 500 ml 0.5% Sodium lauryl sulfate (SLS) in water at 37°C and 50 rpm, at least about 70% of the fenofibrate or the other fibrate drug dissolved in 5 minutes.

The present invention encompasses a composition that comprises a therapeutically effective amount of fenofibrate or another fibrate drug that is dissolved in menthol and further comprises at least one surface active agent that when administered orally to beagle dogs shows a bioavailability of fenofibric acid based on Area Under the Curve (AUC) of the concentration v. time profile in plasma that is at least three times that of the Trichor® 54 mg product on a per milligram basis (when normalized to equal weight).

The present invention also encompasses the method of preparing a composition of the invention, which method comprises:
a) heating menthol to about 60 °C in order to effect melting thereof,
b) adding at least one surface active agent to the melt,
c) cooling the product of step b) to about 50 °C,
d) dissolving fenofibrate or another fibrate drug in the product of step c) with stirring,
e) cooling the product of step d) to room temperature to obtain the composition of the invention, and
f) if capsules are desired, (A) dispensing the product of step e) into capsules, or (B) alternatively adding a solid carrier such as microcrystalline cellulose, lactose or sorbitol, to the product of step e), mixing well, cooling to room temperature and filling the powder thus obtained into capsules.

The present invention encompasses a composition that comprises a therapeutically effective amount of fenofibrate or another fibrate drug that is dissolved in a surfactant mixture, such as Polyethylene Glycol (PEG) 1000 and Poloxamer 407. The composition may be optionally absorbed or adsorbed on a solid carrier.

The present invention encompasses a composition that comprises a therapeutically effective amount of fenofibrate or another fibrate drug that is intimately associated with a surfactant mixture, such as PEG 1000 and Poloxamer 407. This composition can have a dissolution property in that, when tested in 900 ml 0.5% Sodium lauryl sulfate (SLS) in water at 37°C and 50 rpm, at least about 40% of the fenofibrate or the other fibrate drug dissolved in 15 minutes and/or about 80% dissolved in 30 minutes. The intimate association may be in the form of a solution but would encompass compositions where at least part of the drug has come out of such a solution or has not fully dissolved due to e.g. saturation. When administered orally to beagle dogs, this composition of the invention shows a bioavailability of fenofibric acid, based on Area Under the Curve (AUC) of the concentration vs. time profile in plasma, that is at least about two times that of the Trichor® 54 mg product on a per milligram basis (when normalized to equal weight).

The present invention also encompasses a method of treating a patient for elevated triglyceride levels comprising administering to the patient a composition of fenofibrate that comprises a therapeutically effective amount of fenofibrate that is dissolved in menthol.

The present invention also encompasses a method of treating a patient for elevated triglyceride levels comprising administering to the patient a composition of fenofibrate that comprises a therapeutically effective amount of fenofibrate that is dissolved in menthol and further comprises at least one surface active agent.

The present invention also encompasses a method of treating a patient for elevated triglyceride levels comprising administering to the patient a composition of fenofibrate that comprises a therapeutically effective amount of fenofibrate that is dissolved in PEG 1000 and Poloxamer 407.

### Detailed Description of the Invention

There is a need to improve pharmaceutical compositions of fenofibrate, a drug used in treating Hypertriglyceridemia. The term "fenofibrate" includes the 1-methylethyl ester of 2-[4-(4-chlorobenzoyl)-phenoxy]-2-methyl-propanoic acid and any pharmaceutically acceptable salts thereof. One aspect of this invention is to compositions of fenofibrate that is dissolved in menthol. Fenofibrate dissolves up to about 37% in melted menthol at 60°C. Formulations may be made where all the fenofibrate is dissolved in the menthol or where only some of the fenofibrate is so dissolved and the rest present in a solid form in the fully saturated menthol medium. In a preferred embodiment of the invention, the fenofibrate is fully dissolved in the menthol. In one embodiment of the invention, the menthol melt may be filled into capsules in the liquid state or may be solidified, optionally milled, and filled into capsules. The capsules used for the liquid fill in one embodiment may be hard gelatin capsules. In a preferred embodiment, the hard gelatin capsules are banded to prevent leakage. In a more preferred embodiment, the liquid formulation may be filled into soft-gel capsules. In another embodiment, the solidified menthol solution is optionally milled and filled into hard gelatin capsules or equivalent capsules of other materials such as materials of vegetable origin (e.g. HPMC). In another preferred embodiment, the melted menthol formulations may be further adsorbed on a solid carrier. Such solid carriers can be water soluble (hydrosoluble) carriers such as sucrose, lactose, mannitol or sorbitol or water insoluble carriers such as starch, cellulose, microcrystalline cellulose, or calcium phosphate. The so formed powder can optionally be mixed with standard pharmaceutical additives to help flow or other properties and can be filled into hard gelatin capsules or their equivalents. In another preferred embodiment, these powders can be optionally mixed with standard pharmaceutical excipients and formulated for tablet formation in a tablet press.

In this instant patent application, the term "another fibrate drug" or "the other fibrate drug" includes fenofibric acid, any salt of fenofibric acid, any ester of fenofibric acid except the 1-methylethyl ester which is encompassed by the term "fenofibrate" as defined above, bezafibrate, binifibrate, clinofibrate, ciprofibrate, clofibrate, clofibride, etofibrate, etofylline clofibrate, gemfibrozil, pirifibrate, ronifibrate and simfibrate.

In the patent application, where fenofibrate or another fibrate drug is "intimately associated with menthol", "in intimate association with menthol", "intimately associated with a surfactant mixture" or "in intimate association with a surfactant mixture, it is intended to include
a) a solution of fenofibrate or the other fibrate drug in menthol, menthol surfactant mixture, or surfactant mixture whether the menthol, menthol mixture or surfactant mixture is a liquid, melt or solid (a solid solution);
b) a precipitate of fenofibrate or the other fibrate drug, or a co-precipitate of fenofibrate or the other fibrate drug and any additive(s) from the menthol solution, menthol surfactant mixture solution or surfactant mixture solution, which is coated by or in contact with the saturated or supersaturated solution; and/or
c) fenofibrate or the other fibrate drug coated by or in contact with a saturated solution of fenofibrate or the other fibrate drug in menthol, the menthol surfactant mixture or surfactant mixture, wherein the remaining fenofibrate or the other fibrate drug does not dissolve because the amount of fenofibrate or the other fibrate drug present is above saturation.

The expressions, "intimately associated" and "in intimate association", exclude a simple physical mixture of two solids by blending or by granulation in a granulation liquid which does not at least partially dissolve the fenofibrate or the other fibrate drug.

Another aspect of this invention is a composition for the treatment of elevated levels of triglycerides that comprises a therapeutically effective amount of fenofibrate that is dissolved in menthol and further comprises at least one surface active agent. Formulations may be made where all the fenofibrate is dissolved in the menthol or where only some of the fenofibrate is so dissolved and the rest present in a solid form in the fully saturated menthol medium. In a preferred embodiment, the fenofibrate is dissolved in the menthol plus surface active agent medium. In a more preferred embodiment, the fenofibrate is fully dissolved in the menthol which also comprises the surface active agent. Surface active agents that can be used with this embodiment comprise the Tweens, most preferably Tween 80, sodium ducosate, sodium lauryl sulfate, Cremophor, polyethylene glycols (PEG), most preferably PEG 1000, and poloxamers, most preferably poloxamer 407. Preferred embodiments comprise by weight fenofibrate 2% to 40%, more preferably 5% to 25%, menthol 10% to 90%, more preferably 15% to 40%, and surface active agents 10% to 80%, more preferably 30% to 70%. In another preferred embodiment the melted menthol formulations may be further adsorbed on, or absorbed in, a solid carrier. Such solid carriers can be water soluble (hydrosoluble) carriers such as sucrose, lactose or sorbitol or water insoluble carriers such as starch, cellulose, microcrystalline cellulose, or calcium phosphate. The so formed powder can optionally be mixed with standard pharmaceutical additives to help flow or other properties and can be filled into hard gelatin capsules or their equivalents. In another preferred embodiment these powders can be optionally mixed with standard pharmaceutical excipients and formulated for tablet formation in a tablet press or formed into a melt tablet.

In a preferred embodiment of a composition of the invention, a formulation comprised about 25.2% fenofibrate , about 23.4% menthol, about 11.7% sodium ducosate and about 39.7 % Tween 80. When the drug release of this formulation was tested in a small volume drug release test of 50 ml 0.1N HCl at 37°C and 150 rpm, about 11.9% of the fenofibrate in the composition were dissolved in 15 minutes.

Another preferred embodiment of the composition of the invention comprises about 20.5% fenofibrate, about 37.9% menthol, about 9.5% sodium ducosate and about 32.2% Tween 80. When the drug release of this composition was tested in a small volume drug release test of 50 ml 0.1N HCl at 37°C and 150 rpm, about 31.7% of the fenofibrate in the composition were dissolved in 15 minutes.

Another preferred embodiment of the composition of the invention comprises about 12.4% fenofibrate, about 18.4% menthol, and about 69.1 % Tween 80. When the drug release of this composition was tested in a small volume drug release test of 50 ml 0.1N HCl at 37°C and 150 rpm, about 12.5% of the fenofibrate in the composition were dissolved in 15 minutes.

Another preferred embodiment of the composition of the invention comprises about 12.4% fenofibrate, about 18.4% menthol, and about 69.1 % Cremophor. When the drug release of this composition was tested in a small volume drug release test of 50 ml O.1N HCl at 37°C and 150 rpm, about 17.9% of the fenofibrate in the composition were dissolved in 15 minutes.

Another preferred embodiment of the composition of the invention comprises about 10.9% fenofibrate, about 16.2% menthol, about 8.1 % sodium ducosate, about 4.0% glycerine and about 60.7% Cremophor. When the drug release of this composition was tested in a small volume drug release test of 50 ml 0.1N HCl at 37°C and 150 rpm, about 15.6% of the fenofibrate in the composition dissolved in 15 minutes.

In a most preferred embodiment of the composition of the invention comprises about 7.7% fenofibrate, about 19.2% menthol, about 7.7% sodium ducosate and about 65.4% Tween 80. When the drug release of this composition was tested in a small volume drug release test of 50 ml 0.1N HCl at 37°C and 150 rpm, about 93.3% of the fenofibrate in the composition dissolved in 15 minutes. This most preferred embodiment was further tested in 500 ml 0.5% sodium lauryl sulfate (SLS) in water at 37°C and 50 rpm where it gave a release profile of 78.7% dissolved at 5 minutes and 92.5% dissolved at 10 minutes.
By comparison, the fenofibrate composition as taught in example 2 of U.S. application 10/400,100 when tested in less stringent, more conducive conditions for dissolution, in a USP Apparatus II dissolution tester in 900 ml 0.5% sodium lauryl sulfate (SLS) in water at 37°C and 100 rpm, displayed a rate of dissolution such that it took approximately 90 minutes for greater than 90% of the fenofibrate to dissolve. This most preferred embodiment was further tested for its pharmacokinetic profile in dogs vs. the micronized formulation of commercial Trichor® 54 mg (see example 3) and gave a bioavailability of the active metabolite fenofibric acid that was improved by a factor of more than four on a per milligram basis.

Another aspect of this invention encompasses the method of preparing the fenofibrate menthol compositions. In one preferred embodiment, this method comprises the heating of menthol to about 50 - 70°C, most preferably about 60°C, in order to effect melting of the menthol. The menthol melt is stirred at a convenient rate. The method further comprises adding a surface active agent or more than one agent to the melt. The melt is stirred gently until a full solution has been achieved. In a preferred embodiment, the surface active agent is Tween 80. In a more preferred embodiment the surface active agent comprises both Tween 80 and Sodium ducosate. In one embodiment, fenofibrate is added to the melt at this point. In a more preferred embodiment the melt is cooled to between 45°C and 55°C, most preferably to about 50°C, before adding the fenofibrate. The melt is stirred at about the same temperature until all the fenofibrate dissolves. In one preferred embodiment the solution thus obtained is dispensed into either hard or soft capsules. More preferably the solution (or melt) is first cooled to room temperature and then dispensed into either hard or soft capsules. The hard capsules are preferably sealed by "banding" to prevent leakage. In another preferred embodiment of this method a solid carrier such as microcrystalline cellulose, lactose or sorbitol or a combination thereof is added to the melt either before or after cooling to room temperature. The mixture is mixed well, cooled to room temperature if necessary, and filled into capsules. Optionally, other excipients may be added to the powder such as flow aids. In another preferred embodiment the powder so obtained is further formulated with additives that allow it to be pressed into a tablet in a tablet press.

Another aspect of this invention relates to compositions of fenofibrate that are menthol free but comprise a therapeutically effective amount of fenofibrate or other Fibrate drug that is dissolved in polyethylene glycol (PEG) and Poloxamer. PEG useful for this embodiment are all PEG's that are liquid at room temperature or that melt up to about 70°C. The most preferred PEG is PEG 1000. The most preferred Poloxainer is Poloxamer 407. In an embodiment the formulation can comprise by weight fenofibrate from about 5% to about 50%, PEG 1000 from about 5% to about 50% and Poloxamer 407 from about 5% to about 50%. In another preferred embodiment the formulations may be further adsorbed on or absorbed in a solid carrier. Such solid carriers can be water soluble (hydrosoluble) carriers such as sucrose, lactose or sorbitol or water insoluble carriers such as starch, cellulose, microcrystalline cellulose, or calcium phosphate. The so formed powder can optionally be mixed with standard pharmaceutical additives to help flow or other properties and can be filled into hard gelatin capsules or their equivalents. In another preferred embodiment these powders can be optionally mixed with standard pharmaceutical excipients and formulated for tablet formation in a tablet press.

In a preferred embodiment the formulation comprises about 12.4% Fenofibrate, about 18.4% PEG 1000, and about 69.1 % Poloxamer 407. When this embodiment when tested in 900 ml 0.5% Sodium lauryl sulfate (SLS) in water at 37°C and 50 rpm, 79.4% of the fenofibrate were dissolved at 15 minutes, 84.6% were dissolved at 30 minutes and 85.2% were dissolved at 60 minutes. Micronized fenofibrate, tested under the same conditions gave corresponding results of 10.7% for 15 minutes 20.2% for 30 minutes and 31.6% for 60 minutes.

Another preferred embodiment comprises about 35.1 % fenofibrate, about 32.5% PEG 1000, and about 32.5% Poloxamer 407. When this embodiment was when tested in 900 ml 0.5% Sodium lauryl sulfate (SLS) in water at 37°C and 50 rpm, 41.8% of the fenofibrate were dissolved at 15 minutes, 84.9% were dissolved at 30 minutes and 91.8% were dissolved at 60 minutes. Micronized fenofibrate, tested under the same conditions gave corresponding results of 10.7% for 15 minutes 20.2% for 30 minutes and 31.6% for 60 minutes.

A most preferred embodiment comprises about 9.9% fenofibrate, about 6.6% PEG 1000, about 1.0% sodium ducosate, about 6.6% Gelucire® 33/01 and about 9.9% Poloxamer 407, all adsorbed on the solid carrier sorbitol which comprised about 66% of the weight. This preferred embodiment could be delivered in a capsule or more preferably pressed into tablet form. When this embodiment was tested in 500 ml 0.5% Sodium lauryl sulfate (SLS) in water at 37°C and 50 rpm, 18.4% of the fenofibrate were dissolved at 5 minutes, 47.2% were dissolved at 10minutes, 70.9% were dissolved at 20 minutes and 78.0% were dissolved at 30 minutes. This most preferred embodiment of this aspect of the invention was further tested for its pharmacokinetic profile in dogs vs. the micronized formulation of commercial Trichor® 54 mg (see example 3) and gave a bioavailability of the active metabolite fenofibric acid that was more than two times better on a per milligram basis.

Another aspect of this invention encompasses the method of treating a patient for elevated triglyceride levels comprising administering to the patient a composition of fenofibrate that comprises a therapeutically effective amount of fenofibrate that is dissolved in menthol. In one embodiment the drug is dosed as a viscous solution in a capsule. In one embodiment this capsule is a hard gelatin capsule or equivalent. In a preferred embodiment the capsule is sealed by "banding". In another preferred embodiment the capsule is a soft gel capsule of appropriate material. In another preferred embodiment the drug solution is adsorbed on a pharmaceutically acceptable carrier and the drug is dosed as a powder in a capsule and in yet another preferred embodiment the powder is further compounded into a tablet form. In an embodiment of this aspect the composition of fenofibrate is dosed at a level of about 5 mg fenofibrate to 50 mg fenofibrate per day, more preferably about 10 mg to about 40 mg fenofibrate per day and most preferably about 30 to about 35 mg fenofibrate per day.

Another aspect of this invention encompasses the method of treating a patient for elevated triglyceride levels comprising dosing a composition of fenofibrate that comprises a therapeutically effective amount of fenofibrate that is dissolved in menthol and further comprises at least one surface active agent. In one embodiment the composition is dosed as a viscous solution in a capsule. In one embodiment this capsule is a hard gelatin capsule or equivalent. In a preferred embodiment the capsule is sealed by "banding". In another preferred embodiment the capsule is a soft gel capsule of appropriate material. In another preferred embodiment the drug solution is adsorbed on a pharmaceutically acceptable carrier and the drug is dosed as a powder in a capsule and in yet another preferred embodiment the powder is further compounded into a tablet form. In an embodiment of this aspect the composition of fenofibrate is dosed at a level of about 5 mg fenofibrate to 50 mg fenofibrate per day, more preferably about 10mg to about 40 mg fenofibrate per day and most preferably about 30 to about 35 mg fenofibrate per day.

Another aspect of this invention encompasses the method of treating a patient for elevated triglyceride levels comprising dosing a composition of fenofibrate that comprises a therapeutically effective amount of fenofibrate that is dissolved in PEG 1000 and Poloxamer 407. In one embodiment the composition is dosed as a viscous solution in a capsule. In one embodiment this capsule is a hard gelatin capsule or equivalent. In a preferred embodiment the capsule is sealed by "banding". In another preferred embodiment the capsule is a soft gel capsule of appropriate material. In another preferred embodiment the drug solution is adsorbed on a pharmaceutically acceptable carrier and the drug is dosed as a powder in a capsule and in yet another preferred embodiment the powder is further compounded into a tablet form. In an embodiment of this aspect the composition of fenofibrate is dosed at a level of about 10 mg fenofibrate to 100 mg fenofibrate per day, more preferably about 30mg to about 70 mg fenofibrate per day and most preferably about 65 mg fenofibrate per day.

### Examples

### Example 1. Fenofibrate Formulations in Menthol

Formulations were prepared by heating menthol to about 60°C while stirring and adding the additives. The mixture was stirred until all the components dissolved to form a melt. Thereafter, the melt was cooled to about 50°C, fenofibrate was added and stirred until dissolved, the mixture was cooled to room temperature, and dispensed into capsules. The formulations made, on a per capsule basis are listed in Table 1 and Table 2.

**Table 1. Formulations of Fenofibrate in Menthol and Tween**

| **Formulation** | **117.52.2** | **117.52.3** | **117.55.2** | **160.16** |
|---|---|---|---|---|
| Material | mg (%) | mg (%) | mg (%) | mg (%) |
| Fenofibrate | 54 (25.2) | 54 (20.5) | 54 (12.4) | 10 (7.7) |
| Menthol | 50 (23.4) | 100 (37.9) | 80 (18.4) | 25 (19.2) |
| Na Ducosate | 25 (11.7) | 25 (9.5) | 0 | 10 (7.7) |
| Tween 80 | 85 (39.7) | 85 (32.2) | 300 (69.1) | 85 (65.4) |
| **Total** | **214 (100)** | **264 (100)** | **434 (100)** | **130 (100)** |

**Table 2. Formulations of Fenofibrate in Menthol and Cremophor**

| **Formulation** | **117.55.3** | **117.55.6** |
|---|---|---|
| Material | mg (%) | mg (%) |
| Fenofibrate | 54 (12.4) | 54 (10.9) |
| Menthol | 80 (18.4) | 80 (16.2) |
| Na Ducosate | 0 | 40 (8.1) |
| Cremophor | 300 (69.1) | 300 (60.7) |
| Glycerine | 0 | 20 (4.0) |
| **Total** | **434 (100)** | **494 (100)** |

The formulations were tested for their small volume in vitro release characteristics in 50 ml 0.1N HCl at 37°C and 150 rpm using the following procedure:

### III. Instrumentation (or equivalent)

a. Automated Dissolution System comprising of:
   Hanson SR8 Plus Test Station
   Hanson Auto Plus Maximiser System Controller
   Hanson Auto Plus MultiFill Fraction Collector
b. HPLC System comprising of:
   pump - Merck Hitachi L-7100
   autoinjector - Merck Hitachi L-7200
   column oven - Merck Hitachi L-7300
   detector - Merck Hitachi L-7400
   interface and integration software - Merck Hitachi D-7000

### RELEASE TEST PROCEDURE

- Equipment:: 6-vessel assembly, small volume vessels and paddles
- Medium:: 0.1N HCl for 30 min.
- Volume:: 50 ml
- Stirring Rate:: 150 RPM
- Temperature:: 37C 0.5C

### 0.1N HCl Preparation

Dilute 8.5ml HCl 37% to 1 liter with purified water.

### Procedure

Place one weighed capsule in each vessel containing O.1N HCl and immediately operate the apparatus for 30 min.

Unless otherwise specified, 3ml samples are withdrawn from each vessel and immediately filtered through PTFE membranes at 15 and 30 min.

### IV. Analysis Parameters

- Column & Packing:: Hypersil ODS BDS, 150X4.6mm, 5m
- Column Temperature:: RT
- Injector Temperature:: RT
- Mobile Phase:: 40:60 dilute phosphoric acid: acetonitrile
- Flow Rate:: 2.0 ml/min.
- Detector:: UV at 286 nm
- Sample / Injection Volume:: 10 µL
- Injector Wash Solution:: 50:50 purified water: acetonitrile

The small volume was chosen as a model of the conditions in a fasted stomach. The results of these tests are given in Table 3.

**Table 3. In vitro release of Fenofibrate in 50 ml 0.1N HCl**

| **Formulation** | **% dissolved 15 min** | **% dissolved 30 min** |
|---|---|---|
| 117.52.2 | 11.9 | 14.4 |
| 117.52.3 | 31.7 | 32.3 |
| 117.55.2 | 12.5 | 15.7 |
| 160.16 | 93.3 | 83.0 |
| 117.55.3 | 17.9 | 17.5 |
| 117.55.6 | 15.6 | 27.1 |
| Micronized Fenofibrate | 0 | 0 |

Under these conditions micronized fenofibrate gave no dissolution at all. Formulation 160.16 gave the best results under this model condition. The range of values for the 6 tablets were 90.3% to 99.3% at 15 minutes and 74.9% to 94.6% at 30 minutes. The occasional result of lower dissolution at longer time points may be caused by menthol leaching out of the presumed spontaneously formed micelle like structures which are believed to aid in dissolution and the subsequent precipitation of some of the material.

### Example 2. Fenofibrate Formulations in a Surfactant Mixture

Polyethylene glycol (PEG 1000) and poloxamer 407 were heated to 60°C while being stirred. Fenofibrate was added and the stirring continued until all had dissolved. The melt was dispensed into capsules and allowed to cool.

**Table 4. Formulations of Fenofibrate with PEG and Poloxamer**

| **Formulation** | **128.52.2** | **117.58.3** | **Micronized Fenofibrate** |
|---|---|---|---|
| Material | mg (%) | mg (%) | mg (%) |
| Fenofibrate | 54 (12.4) | 54 (35.1) | 54 (100) |
| Menthol | 0 | 0 | 0 |
| Na Ducosate | 0 | 0 | 0 |
| PEG 1000 | 80 (18.4) | 50 (32.5) | 0 |
| Poloxamer 407 | 300 (69.1) | 50 (32.5) | 0 |
| Cremophor | 0 | 0 | 0 |
| Glycerine | 0 | 0 | 0 |
| **Total** | **434 (100)** | **154 (100)** | **54 (100)** |

These two formulations were tested, along with untreated micronized fenofibrate, for their in vitro release in a USP type II dissolution tester using 900 ml water containing 0.5% SLS at 37°C and 50 rpm. Fenofibrate content of the solutions was determined by HPLC as described above. The results are shown in Table 5

**Table 5. Cumulative Release of Fenofibrate in 900 ml 0.5% SLS**

| **Formulation** | Micronized Fenofibrate | 128.52.2 | 117.58.3 |
|---|---|---|---|
| Time (min) | % released | % released | % released |
| 15 | 10.7 | 79.4 | 41.8 |
| 30 | 20.2 | 84.6 | 84.9 |
| 60 | 31.6 | 85.2 | 91.8 |

### Example 3. In Vivo Pharmacokinetics in Dogs

Two formulations were prepared as shown in Table 6. MAZ118 has the same formulation as 160.16 in Example 1. Formulation 107.69 is based on PEG 1000, poloxamer 407 as the formulations in Example 2 with the addition of a small amount (1%) of sodium ducosate and sorbitol as a solid carrier. In both cases the fenofibrate is in solution in the formulation.

**Table 6. Formulations used in the trial**

| **Formulation** | **MAZ118** | **107.69** |
|---|---|---|
| Material | mg (%) | mg (%) |
| Fenofibrate | 10 (7.7) | 9.9 (9.9) |
| Menthol | 25 (19.2) | 0 |
| Na Ducosate | 10 (7.7) | 1.0 (1.0) |
| Tween 80 | 85 (65.4) | 0 |
| Gelucire 33/01 | 0 | 6.6 (6.6) |
| PEG 1000 | 0 | 6.6 (6.6) |
| Poloxamer 407 | 0 | 9.9 (9.9) |
| sorbitol | 0 | 66.0 (66.0) |
| **Total** | **130 (100)** | **100 (100)** |

### Production of MAZ118

A double walled glass reactor was heated to 65°C. Menthol (EP), 50 grams, Tween 80 ( Uniqema), 170 grams, and Ducosate Sodium (USP), 20 grams were added to the reactor. The mixture was stirred at 200 rpm until a melt solution was formed. Fenofibrate (Chemagis Ltd.), 20 grams, was added to the above melt and stirred at 200 rpm until full dissolution took place. The solution was cooled to 30°C. Capsules size "0" were filled with the melt solution, 130 mg ± 7 mg. The solution cooled to a viscous liquid. The capsules were found to have 10.5 mg ± 3.9% RSD of fenofibrate in a viscous liquid.

### Production of 107.69

A glass reactor was heated to 65°C. Gelucire 33/01(Gattefosse), 1.0 grams, PEG 1000 (NF), 1.0 gram, Poloxamer 407 (BASF), 1.5 gram, and Ducosate Sodium (USP), 1.0 gram, were added to the reactor. The mixture was stirred at ~200 rpm until a melt solution was formed. Fenofibrate (Chemagis Ltd.), 1.5 grams, was added to the above melt and stirred at ~200 rpm until full dissolution took place. To the melt was added Sorbitol (NF), 10.0 grams , the mixture was mixed well and cooled. Tablets of 7 mm diameter and weighing 100 mg each were hand pressed in a Manesty F3 single punch tablet press. Each tablet had 9.9 mg of fenofibrate.

In vitro dissolution of these two formulations along with formulation 160.16 were carried out as follows:
- Equipment:: 6-vessel assembly, Apparatus 2/II (Paddle).
- Medium:: 0.5% SLS for 1 hour
- Volume:: 500 ml
- Stirring Rate:: 50 RPM
- Temperature:: 37°C±0.5°C
The fenofibrate content of the samples was determined by HPLC as above.
The results of the dissolution test are shown in Table 7.

**Table 7. % Cumulative Dissolution of Fenofibrate**

| **Formulation** | 160.16 | | MAZ118 | | 107.69 | |
|---|---|---|---|---|---|---|
| Time (min) | % released | % released range (n=6) | % released | % released range (n=12) | % released | % released range (n=6) |
| 5 | 78.7 | 58.0 - 96.4 | n.m. | n.m. | 18.4 | 14.3 - 20.0 |
| 10 | 92.5 | 88.8-106.0 | n.m. | n.m. | 47.2 | 44.3 - 50.4 |
| 15 | n.m. | n.m. | 104.5 | 83.6 - 126.6 | n.m. | n.m |
| 20 | 93.5 | 83.3-102.4 | n.m | n.m | 70.9 | 67.5 - 76.5 |
| 30 | 96.7 | 89.9-106.9 | 104.5 | 98.0 - 112.7 | 78.0 | 73.0 - 80.4 |
| 60 | 94.2 | 87.5-103.7 | 105.0 | 99.0 - 112.4 | 82.5 | 79.8 - 86.4 |

| | | | | | | |
|---|---|---|---|---|---|---|
| n.m. = not measured | | | | | | |

### PK Trial in Dogs

The trial was conducted as an open-label, randomized, single-dose, 3-way crossover comparative bioavailability study. The study was designed to determine the AUC₀₋ₜ, AUC_{inf}, Cₘₐₓ, Tₘₐₓ and t_{½} for each formulation. The sample group consisted of six beagle dogs (five female and one male weighing about 10 kg each). Each dog was administered one of three treatments. The first treatment, treatment A, comprised of administering a hard gelatin capsule containing 10 mg fenofibrate formulation MAZ118; the second treatment, treatment B, comprised administration of a 1 x 54 mg fenofibrate tablet Trichor® (Abbott Laboratories); and the third treatment, treatment C, comprised of administration of a hard gelatin capsule containing 10 mg fenofibrate formulation 107.69. Each dog was administered a single oral dose with 10 ml water. After a two week washout the dogs were crossed over to another of the treatments.

Blood samples (4 ml) were collected in EDTA containing tubes before dosing and at 0.5, 1, 1.5, 2, 3, 4, 6, 8, and 24 hours post dosing. The samples were analyzed for fenofibric acid in plasma using an LC/MS/MS method validated for the range of 5 to 100 ng/ml.

### Results

The individual and average pharmacokinetic parameters for each of the two test sessions (A and C) compared to the reference session (B) are given in Tables 8 and 9.

**Table 8. Individual and Average Pharmacokinetic Parameters for Fenofibric Acid in Plasma for the Comparison of 10 mg MAZ118 to 54 mg Trichor®**

| | RESULTS OF | Fenofibrate dog data-final | | dose test (mg)= | 10 | MAZ118 | |
|---|---|---|---|---|---|---|---|
| | | | | dose ref (mg)= | 54 | Trichor | |
| vol-sess | AUC (h*ng/g) | AUCinf (h*ng/g) | T_{½} (h) | Tₘₐₓ (h) | Cmax (ng/g) | **Cmaxtest/ Cmaxref** | **AUCinftest / AUCinfref** |
| dog #1 (test) | 4670.7 | 4878.2 | 6.0 | 0.5 | 1068.2 | 1.26 | 0.90 |
| dog #2 (test) | 3413.9 | 4808.0 | 14.1 | 0.5 | 978.2 | 1.97 | 1.11 |
| dog #4 (test) | 4566.1 | 6232.3 | 15.0 | 1.0 | 1229.4 | 0.50 | 0.81 |
| dog #5 (test) | 5187.8 | 7245.2 | 16.8 | 1.0 | 2122.0 | 1.26 | 0.73 |
| dog #9 (Test) | 4041.0 | 5035.5 | 11.0 | 1.5 | 795.5 | 1.43 | 0.94 |
| dog #12 (test) | 7661.6 | 9588.4 | 11.3 | 1.0 | 1872.8 | 0.98 | |
| | | | | | | | |
| dog #1 (ref) | 4839.8 | 5394.7 | 7.3 | 0.5 | 847.7 | | |
| dog #2 (ref) | 3143.8 | 4321.6 | 13.4 | 0.5 | 497.4 | | |
| dog #4 (ref) | 6196.4 | 7698.1 | 12.0 | 0.5 | 2479.2 | | |
| dog#5 (ref) | 6745.4 | 9950.7 | 17.6 | 1.5 | 1688.3 | | |
| dog#9 (ref) | 3563.6 | 5367.5 | 16.8 | 0.5 | 554.9 | | |
| dog#12 (ref) | 9811.3 | | | 1.0 | 1917.8 | | |
| | | | | | | | |
| **AVG(test)** | **4923.5** | **6297.9** | **12.3** | **0.9** | **1344.3** | **1.232** | **0.899** |
| **AVG (ref)** | **5716.7** | **6546.5** | **13.4** | **0.8** | **1330.9** | | |
| | | | | | | | |
| **%CV (test)** | **29.87%** | **29.75%** | **31.04%** | **41.06%** | **39.48%** | | |
| **%CV (ref)** | **42.90%** | **34.65%** | **30.66%** | **55.78%** | **61.24%** | | |

**Table 9. Individual and Average Pharmacokinetic Parameters for Fenofibric Acid in Plasma for the Comparison of 10 mg Formulation 107.69 to 54 mg Trichor®**

| | RESULTS OF | Fenofibrate dog data | | dose test (mg)= | 10 | 107.69 | |
|---|---|---|---|---|---|---|---|
| | | | | dose ref (mg) = | 54 | Trichor | |
| vol-sess | AUC (h*ng/g) | AUCinf (h*ng/g) | T_{½} (h) | Tₘₐₓ (h) | Cmax (ng/g) | **Cmaxtest/ Cmaxref** | **AUCinftest/ AUCinfref** |
| dog #1 (test) | 2224.0 | | | 0.5 | 441.8 | 0.52 | |
| dog #2 (test) | 2250.2 | 2598.7 | 9.4 | 0.5 | 508.4 | 1.02 | 0.60 |
| dog #4 (test) | 2918.4 | 3392.2 | 10.3 | 0.5 | 1019.3 | 0.41 | 0.44 |
| dog #5 (test) | 3242.3 | 3816.7 | 10.2 | 0.5 | 732.2 | 0.43 | 0.38 |
| dog #9 (Test) | 2070.6 | 2436.3 | 9.6 | 0.5 | 471.9 | 0.85 | 0.45 |
| dog #12 (test) | 2915.7 | 4163.4 | 15.0 | 1.0 | 523.9 | 0.27 | |
| | | | | | | | |
| dog #1 (ref) | 4839.8 | 5394.7 | 7.3 | 0.5 | 847.7 | | |
| dog #2 (ref) | 3143.8 | 4321.6 | 13.4 | 0.5 | 497.4 | | |
| dog #4 (ref) | 6196.4 | 7698.1 | 12.0 | 0.5 | 2479.2 | | |
| dog#5 (ref) | 6745.4 | 9950.7 | 17.6 | 1.5 | 1688.3 | | |
| dog#9 (ref) | 3563.6 | 5367.5 | 16.8 | 0.5 | 554.9 | | |
| dog#12 (ref) | 9811.3 | | | 1.0 | 1917.8 | | |
| | | | | | | | |
| **AVG(test)** | **2603.5** | **3281.5** | **10.9** | **0.6** | **616.2** | **0.585** | **0.470** |
| **AVG (ref)** | **5716.7** | **6546.5** | **13.4** | **0.8** | **1330.9** | | |
| | | | | | | | |
| **%CV (test)** | **18.48%** | **22.89%** | **21.56%** | **34.99%** | **36.09%** | | |
| **%CV (ref)** | **42.90%** | **34.65%** | **30.66%** | **55.78%** | **61.24%** | | |

Table 8 shows that the average AUC₀₋ₜ for MAZ118 was 4923 (ng*hr/ml) or 492 (ng*hr/ml) per mg while the average AUC₀₋ₜ for Trichor® was 5716 (ng*hr/ml) or 106 (ng*hr/ml) per mg. The bioavailability of the 10 mg formulation as expressed by AUC was 86% that of the 54 mg Trichor®. On a per mg basis the MAZ118 test formulation was 4.6 times more available. The corresponding values for AUC_{inf} for the samples where a terminal half life (t_{½}) could be calculated showed the MAZ118 test to be 5.2 times as effective as the Trichor® on a per milligram basis (630 (ng*hr/ml) per mg compared to 121 (ng*hr/ml) per mg). The average of the ratios of the individual AUC_{inf} shows the 10 mg test formulation MAZ118 to have 90% of the bioavailability of the 54 mg Trichor®. The average Cₘₐₓ for MAZ118 was 1344.3 (ng/ml) or 134 (ng /ml) per mg while the average Cₘₐₓ for Trichor® was 1330.9 (ng/ml) or 24.6 (ng/ml) per mg. The values for average Tₘₐₓ were similar, 0.9 hr for the test formulation and 0.8 hr for the reference. The terminal elimination half life was similar for each formulation being 12.3 hours for the test formulation and 13.4 hours for Trichor® reference formulation. The variability of the test formulation MAZ118, as expressed by %CV, was lower than in the reference formulation for both the AUC parameters and the Cmax parameter.

Table 9 shows that the average AUC₀₋ₜ for formulation 107.69 was 2603 (ng*hr/ml) or 260 (ng*hr/ml) per mg while the average AUC₀₋ₜ for Trichor® was 5716 (ng*hr/ml) or 106 (ng*hr/ml) per mg. The bioavailability of the 10 mg formulation as expressed by AUC was 46% that of the 54 mg Trichor®. On a per mg basis the 107.69 test formulation was 2.4 times more available. The corresponding values for AUC_{inf} for the samples where a terminal half life (t_{½}) could be calculated showed the 107.60 test to be 2.7 times as effective as the Trichor® on a per milligram basis (328 (ng*hr/ml) per mg compared to 121 (ng*hr/ml) per mg). The average of the ratios of the individual AUC_{inf} shows the 10 mg test formulation 107.69 to have 47% of the bioavailability of the 54 mg Trichor®. The average Cₘₐₓ for formulation 107.69 was 616.2 (ng/ml) or 62 (ng /ml) per mg while the average Cₘₐₓ for Trichor® was 1330.9 (ng/ml) or 24.6 (ng/ml) per mg. The values for average Tₘₐₓ were similar, 0.6 hr for the test formulation and 0.8 hr for the reference. The terminal elimination half life was similar for each formulation being 10.9 hours for the test formulation and 13.4 hours for Trichor® reference formulation. The variability of the test formulation 107.69, as expressed by %CV, was lower than in the reference formulation for all the PK parameters measured.

### Conclusion:

Both test formulations were shown to be more bioavailable than the reference on a per milligram basis. Formulation 107.69, a solublized form of fenofibrate, was about 2.5 times more bioavailable as the reference. Formulation MAZ118, a solublized form of fenofibrate comprising menthol and a surfactant, was about 5 times as bioavailable on a per milligram basis.
Further aspects and features of the present inventions are set out in the following numbered clauses.
1. A composition comprising fenofibrate or another fibrate drug, and at least one pharmaceutical excipient, wherein the composition has a dissolution of (a) at least about 80% in about 15 minutes, as measured using a rotating blade method at 150 rpm, in 50 ml of dissolution medium composed of 0.1N HCl at 37°C; or (b) at least about 90% in about 15 minutes, as measured using a rotating blade method at 50 rpm, in 500 ml of a dissolution medium constituted by water with 0.5% sodium lauryl sulfate at 37°C
2. A composition comprising fenofibrate or another fibrate drug, which when administered to a dog weighing about 10 kg (a) at a dose of about 10 mg presents an average AUC₀₋ₜ of fenofibric acid about 4923 (ng*hr/ml); (b) presents an average AUC₀₋ₜ of fenobric acid of about 492 (ng*hr/ml) per mg or (c) at a dose of about 10 mg presents a Cmax of fenofibric acid of at least about 800 ng/ml and an average Cmax of about 1300 ng/ml.
3. The composition of any one of clauses 1 to 2 wherein the fenofibrate or other fibrate drug is in intimate association with menthol.
4. The composition of clause 3 which further comprises at least one surface active agent.
5. The composition of clause 4 wherein the at least one surface active agent is selected from Cremophor, sodium ducosate and Tween 80, preferably sodium ducosate and Tween 80.
6. The composition of any of clause 5, consisting of about 7.7% fenofibrate, about 19.2% menthol, about 7.7% sodium ducosate and about 65.4% Tween80.
7. The composition of clause 6, wherein when the composition is tested in (a) a small volume drug release test of 50 ml 0.1N HCl at 37°C and 150 rpm, at least about 90% of the fenofibrate in the composition are dissolved in 15 minutes; (b) a paddle method with 500 ml 0.5% sodium lauryl sulfate in water at 37°C and 50 rpm, at least about 75% of the fenofibrate in the composition are dissolved in 5 minutes; or (c) a paddle method with 500 ml 0.5% sodium lauryl sulfate in water at 37°C and 50 rpm, at least about 90% of the fenofibrate in the composition are dissolved in 10 minutes.
8. The composition of clause 5, comprising about 25.2% fenofibrate, about 23.4% menthol, about 11.7% sodium ducosate and about 39.7% Tween 80.
9. The composition of clause 8, wherein when the composition is tested in a small volume drug release test of 50 ml 0.1N HCl at 37°C and 150 rpm, at least about 10% of the fenofibrate in the composition are dissolved in 15 minutes.
10. The composition of clause 5, comprising about 20.5% fenofibrate, about 37.9% menthol, about 9.5% sodium ducosate and about 32.2% Tween 80.
11. The composition of clause 10, wherein when the composition is tested in a small volume drug release test of 50 ml 0.1N HCl at 37°C and 150 rpm, at least about 30% of the fenofibrate in the composition are dissolved in 15 minutes.
12. The composition of clause 5, comprising about 12.4% fenofibrate, about 18.4% menthol and about 69.1 % Tween 80.
13. The composition of clause 12, wherein when the composition is tested in a small volume drug release test of 50 ml 0.1N HCl at 37°C and 150 rpm, at least about 10% of the fenofibrate in the composition are dissolved in 15 minutes.
14. The composition of clause 5, comprising about 12.4% fenofibrate, about 18.4% menthol and about 69.1 % Cremophor.
15. The composition of clause 14, wherein when the composition is tested in a small volume drug release test of 50 ml O.1N HCl at 37°C and 150 rpm, at least about 15% of the fenofibrate in the composition are dissolved in 15 minutes.
16. The composition of clause 5, comprising about 10.9% fenofibrate, about 16.2% menthol, about 8.1 % sodium ducosate, about 60.7% Cremophor and about 4.0% glycerine.
17. The composition of clause 16, wherein when the composition is tested in a small volume drug release test of 50 ml 0.1N HCl at 37°C and 150 rpm, at least about 15% of the fenofibrate in the composition are dissolved in 15 minutes.
18. A composition comprising fenofibrate, menthol and at least one surface active agent.
19. The composition of clause 18, wherein the composition has a dissolution of (a) at least about 10% in about 15 minutes, as measured using a rotating blade method at 150 rpm, in 50 ml of dissolution medium composed of 0.1N HCl at 37°C; (b) at least about 30% in about 15 minutes, as measured using a rotating blade method at 150 rpm, in 50 ml of dissolution medium composed of 0.1N HCl at 37°C;(c) at least about 80% in about 15 minutes, as measured using a rotating blade method at 150 rpm, in 50 ml of dissolution medium composed of 0.1N HCl at 37°C; or (d) between at least about 10% to at least about 80% in about 15 minutes, as measured using a rotating blade method at 150 rpm, in 50 ml of dissolution medium composed of 0.1N HCl at 37°C.
20. The composition of any preceding clause, wherein the fenofibrate and menthol are in intimate association.
21. The composition of any of clauses 18 to 20 comprising, in intimate association, fenofibrate, menthol, sodium ducosate and Tween 80, wherein the fenofibrate is at least partially dissolved in said menthol.
22. The composition of any of clauses 18 to 21, wherein the composition is in the form of a solution.
23. The composition of any of clauses 18 to 21, wherein the composition is adsorbed on a pharmaceutically acceptable carrier.
24. The composition of clause 23, wherein the pharmaceutically acceptable carrier is chosen from sucrose, lactose, sorbitol, mannitol, starch, cellulose, microcrystalline cellulose and calcium phosphate.
25. A method of preparing a fenofibrate composition, comprising mixing at least one surface active agent and fenofibrate in melted menthol until at least some of the fenofibrate dissolves.
26. The method of clause 25, further comprising dispensing the composition into capsules.
27. The method of clause 26, wherein the capsules are hard gelatin capsules or equivalent capsules of vegetable origin.
28. The method of clause 26 or 27, wherein the capsules are further sealed by banding.
29. The method of clause 26, wherein the capsules are soft gel capsules.
30. The method of any of clauses 25 to 29, wherein the menthol melt mixture is further adsorbed onto a pharmaceutically acceptable carrier.
31. The method of clause 30, wherein the pharmaceutically acceptable carrier is chosen from a group consisting of sucrose, lactose , sorbitol , mannitol, starch, cellulose, microcrystalline cellulose, calcium phosphate and mixtures thereof.
32. The method of clause 30 or 31, wherein the composition is filled into capsules or further processed into tablets.
33. A composition comprising fenofibrate or another fibrate drug in intimate association with a surfactant mixture.
34. The composition of clause 33, wherein the surfactant mixture comprises a polyethylene glycol and a poloxamer.
35. The composition of clause 33, wherein the composition has a dissolution of at least about 80% in about 30 minutes, as measured using a USP type II dissolution tester using 900 ml water containing 0.5% sodium lauryl sulfate at 37°C and 50 rpm.
36. The composition of clause 33 or 34, wherein the polyethylene glycol is PEG 1000 and the poloxamer is poloxamer 407.
37. The composition of any of clauses 33 to 35 comprising (a) about 12.4% fenofibrate, about 18.4% PEG 1000, and about 69.1 % poloxamer 407; (b) about 35.1 % fenofibrate, about 32.5% PEG 1000, and about 32.5% poloxamer 407; or (c) about 9.9% fenofibrate, about 6.6% PEG 1000, about 9.9% poloxamer 407, about 1.0% sodium ducosate, about 6.6% Gelucire (33/01) and about 66.0% sorbitol, wherein the fenofibrate, PEG 1000, poloxamier 407, sodium ducosate and Gelucire are adsorbed on the sorbitol.
38. The composition of any of clauses 33 to 36, wherein when the composition is administered to a dog weighing about 10 kg (a) at a dose of about 10 mg, an average AUC₀₋ₜ of fenofibric acid of about 2604 (ng*hr/ml) is achieved; (b) an average AUC₀₋ₜ of fenofibric acid of about 260 (ng*hr/ml) per mg is achieved; or (c) at a dose of about 10 mg, a Cmax of fenofibric acid of at least about 400 ng/ml and an average Cmax of about 600 ng/ml are achieved.
39. Use of a composition of any of clauses 1 to 24 or 33 to 38, or the product of any of clauses 25 to 32, for the manufacture of a medicament for treating an elevated triglyceride level in a patient.
40. Use clause 39, wherein a fenofibrate dose of 5 to 50 mg per day is administered.

## Claims

1. A composition comprising fenofibrate, menthol and at least one surface active agent.

2. The composition of any preceding claim, wherein the at least one surface active agent is selected from Cremophor, sodium ducosate and Tween 80, preferably sodium ducosate and Tween 80.

3. The composition of claim 2, wherein the at least one surface active agent comprises sodium ducosate and Tween 80.

4. The composition of any preceding claim, wherein the composition is adsorbed on a pharmaceutically acceptable carrier.

5. The composition of claim 4, wherein the pharmaceutically acceptable carrier is chosen from sucrose, lactose, sorbitol, mannitol, starch, cellulose, microcrystalline cellulose and calcium phosphate.

6. The composition of any preceding claim, comprising about 7.7% fenofibrate, about 19.2% menthol, about 7.7% sodium ducosate and about 65.4% Tween80.

7. The composition of claim 6, wherein when the composition is tested in (a) a small volume drug release test of 50 ml 0.1N HCl at 37°C and 150 rpm, at least about 90% of the fenofibrate in the composition are dissolved in 15 minutes; (b) a paddle method with 500 ml 0.5% sodium lauryl sulfate in water at 37°C and 50 rpm, at least about 75% of the fenofibrate in the composition are dissolved in 5 minutes; or (c) a paddle method with 500 ml 0.5% sodium lauryl sulfate in water at 37°C and 50 rpm, at least about 90% of the fenofibrate in the composition are dissolved in 10 minutes.

8. The composition of any of claims 1 to 5, comprising about 25.2% fenofibrate, about 23.4% menthol, about 11.7% sodium ducosate and about 39.7% Tween 80.

9. The composition of claim 8, wherein when the composition is tested in a small volume drug release test of 50 ml 0.1N HCl at 37°C and 150 rpm, at least about 10% of the fenofibrate in the composition are dissolved in 15 minutes.

10. The composition of any of claims 1 to 5, comprising about 20.5% fenofibrate, about 37.9% menthol, about 9.5% sodium ducosate and about 32.2% Tween 80.

11. The composition of claim 10, wherein when the composition is tested in a small volume drug release test of 50 ml 0.1N HCl at 37°C and 150 rpm, at least about 30% of the fenofibrate in the composition are dissolved in 15 minutes.

12. The composition of any of claims 1, 2, 4 and 5, comprising about 12.4% fenofibrate, about 18.4% menthol and about 69.1% Tween 80.

13. The composition of claim 12, wherein when the composition is tested in a small volume drug release test of 50 ml 0.1N HCl at 37°C and 150 rpm, at least about 10% of the fenofibrate in the composition are dissolved in 15 minutes.

14. The composition of any of claims 1, 2, 4 and 5, comprising about 12.4% fenofibrate, about 18.4% menthol and about 69.1% Cremophor.

15. The composition of claim 14, wherein when the composition is tested in a small volume drug release test of 50 ml 0.1N HCl at 37°C and 150 rpm, at least about 15% of the fenofibrate in the composition are dissolved in 15 minutes.

16. The composition of any of claims 1, 2, 4 and 5, comprising about 10.9% fenofibrate, about 16.2% menthol, about 8.1 % sodium ducosate, about 60.7% Cremophor and about 4.0% glycerine.

17. The composition of claim 16, wherein when the composition is tested in a small volume drug release test of 50 ml 0.1N HCl at 37°C and 150 rpm, at least about 15% of the fenofibrate in the composition are dissolved in 15 minutes.

18. The composition of any of claims 1 to 5, wherein the composition has a dissolution of (a) at least about 10% in about 15 minutes, as measured using a rotating blade method at 150 rpm, in 50 ml of dissolution medium composed of 0.1N HCl at 37°C; (b) at least about 30% in about 15 minutes, as measured using a rotating blade method at 150 rpm, in 50 ml of dissolution medium composed of 0.1N HCl at 37°C; or (c) between at least about 10% to at least about 80% in about 15 minutes, as measured using a rotating blade method at 150 rpm, in 50 ml of dissolution medium composed of 0.1N HCl at 37°C.

19. A composition comprising fenofibrate and menthol wherein the fenofibrate and menthol are in intimate association.

20. The composition of any of claims 1 to 5 and 19, wherein the composition has a dissolution of (a) at least about 80% in about 15 minutes, as measured using a rotating blade method at 150 rpm, in 50 ml of dissolution medium composed of 0.1N HCl at 37°C; or (b) at least about 90% in about 15 minutes, as measured using a rotating blade method at 50 rpm, in 500 ml of a dissolution medium constituted by water with 0.5% sodium lauryl sulfate at 37°C

21. The composition of any of claims 1 to 5 and 19, which when administered to a dog weighing about 10 kg (a) at a dose of about 10 mg presents an average AUC₀₋ₜ of fenofibric acid about 4923 (ng*hr/ml); (b) presents an average AUC₀₋ₜ of fenobric acid of about 492 (ng*hr/ml) per mg or (c) at a dose of about 10 mg presents a Cmax of fenofibric acid of at least about 800 ng/ml and an average Cmax of about 1300 ng/ml.

22. The composition of any of claims 1 to 18, wherein the fenofibrate and menthol are in intimate association.

23. The composition of claim 22 wherein the fenofibrate is at least partially dissolved in said menthol.

24. The composition of claim 22, wherein the composition is in the form of a solution.

25. A method of preparing a fenofibrate composition, comprising mixing at least one surface active agent and fenofibrate in melted menthol until at least some of the fenofibrate dissolves.

26. The method of claim 25, further comprising dispensing the composition into capsules.

27. The method of claim 26, wherein the capsules are hard gelatin capsules or equivalent capsules of vegetable origin.

28. The method of claim 26 or claim 27, wherein the capsules are further sealed by banding.

29. The method of claim 26, wherein the capsules are soft gel capsules.

30. The method of any of claims 25 to 29, wherein the menthol melt mixture is further adsorbed onto a pharmaceutically acceptable carrier.

31. The method of claim 30, wherein the pharmaceutically acceptable carrier is chosen from a group consisting of sucrose, lactose, sorbitol, mannitol, starch, cellulose, microcrystalline cellulose, calcium phosphate and mixtures thereof.

32. The method of claim 30 or 31, wherein the composition is filled into capsules or further processed into tablets.

33. A composition comprising fenofibrate or another fibrate drug in intimate association with a surfactant mixture.

34. The composition of claim 33, wherein the surfactant mixture comprises polyethylene glycol and a poloxamer.

35. The composition of claim 34, wherein the composition has a dissolution of at least about 80% in about 30 minutes, as measured using a USP type II dissolution tester using 900 ml water containing 0.5% sodium lauryl sulfate at 37°C and 50 rpm.

36. The composition of any of claims 33 to 35, wherein the polyethylene glycol is PEG 1000 and the poloxamer is poloxamer 407.

37. The composition of any of claims 33 to 36 comprising (a) about 12.4% fenofibrate, about 18.4% PEG 1000, and about 69.1% poloxamer 407; (b) about 35.1% fenofibrate, about 32.5% PEG 1000, and about 32.5% poloxamer 407; or (c) about 9.9% fenofibrate, about 6.6% PEG 1000, about 9.9% poloxamer 407, about 1.0% sodium ducosate, about 6.6% Gelucire (33/01) and about 66.0% sorbitol, wherein the fenofibrate, PEG 1000, poloxamier 407, sodium ducosate and Gelucire are adsorbed on the sorbitol.

38. The composition of any of claims 33 to 37 wherein when the composition is administered to a dog weighing about 10 kg (a) at a dose of about 10 mg, an average AUC₀₋ₜ of fenofibric acid of about 2604 (ng*hr/ml) is achieved; (b) an average AUC₀₋ₜ of fenofibric acid of about 260 (ng*hr/ml) per mg is achieved; or (c) at a dose of about 10 mg, a Cmax of fenofibric acid of at least about 400 ng/ml and an average Cmax of about 600 ng/ml are achieved.

39. Use of a composition of any of claims 1 to 24 or 33 to 38 or a product of any of claims 25 to 32 for the manufacture of a medicament for treating an elevated triglyceride level in a patient.

40. The use of claim 38, wherein a fenofibrate dose of 5 to 50 mg per day is administered.
